# EUROPEAN PATENT SPECIFICATION

(11) **EP 1 515 681 B1**
(45) Date of publication and mention of the grant of the patent: **24.09.2008**
(21) Application number: 03732900.0
(22) Date of filing: 12.06.2003
(51) Int. Cl.: A61Q 13/00, A61Q 15/00, A61K 8/11, A61K 8/73, A23L 1/22

(54) **NON-CRYSTALLINE PERFUME OR FLAVOUR DELIVERY SYSTEM**
NICHT-KRISTALLINES RIECH- UND AROMASTOFFFREISETZUNGSSYSTEM
SYSTEME DE LIBERATION DE PARFUM OU D'AROME NON CRISTALLIN

(30) Priority: 14.06.2002 WO PCT/IB02/02228
(43) Date of publication of application: 23.03.2005
(73) Proprietor: FIRMENICH SA, 1211 Genève 8 (CH)
(72) Inventor: NORMAND, Valéry, 74490 St-Jeoire-en-Faucigny (FR); CANTERGIANI, Ennio, 1807 Blonay (CH); BOUQUERAND, Pierre-Etienne, F-74930 Pers-Jussy (FR); BARRA, Jérôme, F-74160 Neydens (FR); BENCZEDI, Daniel, CH-1228 Plan-Les-Ouates (CH)
(74) Representative: Schneiter, Sorin
(86) International application number: PCT/IB2003/002592
(87) International publication number: WO 2003/105786

(56) References cited:
- EP-A- 0 913 406
- WO-A-01/35764
- WO-A-02/09663
- WO-A-02/47492
- WO-A-99/55819
- WO-A-02/065858
- GB-A- 1 071 170
- US-A- 5 656 584

## Description

### Technical field and prior art

The present invention relates to the perfume and flavour industries. It concerns more particularly an encapsulation system capable of releasing a hydrophobic perfume or flavour ingredient or composition which is usually crystalline at ambient pressure and temperature, in a non-crystalline form, thus providing an advantageous delivery system.

Encapsulation is widely used to provide delivery systems for flavour or fragrance substances known to be volatile and labile. The flavour industry for instance is well fitted with a rich literature, in particular patents, related to encapsulation processes for the preparation of encapsulated flavouring ingredients or compositions. This industry constantly seeks improvements for such processes and for the products there-obtained, in terms of increase of the flavour retention, or of better control of the release of active ingredients from the finished products.

Many methods for the preparation of delivery systems are disclosed in the literature. Extrusion is in particular widely described. During the preparation of an extruded encapsulation system, the ingredient or composition to be protected is typically admixed with a carrier material and the blend thus formed is then heated in order to form a molten mass which is extruded and further cooled and solidified. The physical states of both the carrier material and the active ingredient to be protected are obviously affected by the process and more particularly by the changes in temperatures occurring during the respective heating and cooling steps.

In the flavour industry, the physical state of delivery systems is a fundamental parameter often referred to. A solid state characterised by extremely low molecular mobility is most of the time required to stabilise flavour molecules in an encapsulated dosage form. The solid carrier material used in the delivery systems is usually in an amorphous glassy state, i.e. in a non crystalline form at ambient temperature. In fact, the oligomeric or polymeric molecules on which a carrier material is normally based, are unable to crystallise for kinetic reasons at the end of an encapsulation process. The concept of glass transition temperature (Tg) is well described in the prior art. It represents the transition temperature from a rubbery liquid state to a glassy solid state : such a transition is characterised by a rapid increase in viscosity over several orders of magnitude and over a rather small temperature range. It is recognised by many experts in the field that, in the glassy state, i.e. at temperatures below Tg, all molecular diffusion is highly restricted and it is this process which provides such effective entrapping of the volatile flavours or perfumes, as well preventing other chemical events such as oxidation from occurring. Implicit in much of the literature is the converse, namely that at temperatures above Tg, the encapsulation of flavour or perfume molecules will be ineffective, hence the importance of creating polymeric encapsulating materials with Tg values above ambient temperature.

In the prior art related to perfume or flavour encapsulation, the physical state always referred to, is that of the encapsulating carrier material, which is related to the physical state desired for a suitable handling of the final product. There is no document in the prior art where reference is made to the physical state of the encapsulated material within the delivery system. The literature in this field describes above all the encapsulation of liquid substances which are by nature, amorphous. On the other hand, there is very few literature regarding the encapsulation of solid crystalline ingredients. WO 00/25606 discloses an extruded delivery system, suitable for the delivery of hydrophilic flavouring or perfuming ingredients, among which solid compounds are considered. In the systems there-disclosed, both the carrier material and the active ingredient to be encapsulated are hydrophilic. On the other hand, the literature is silent about the encapsulation of hydrophobic crystalline ingredients. However, the "incompatibility" between conventional hydrophilic carrier materials and hydrophobic crystalline ingredients would tend to prove that a solid hydrophobic ingredient would still be in a crystalline state when incorporated in such a carrier.

Now, in a totally unexpected manner, we have been able to prepare a novel delivery system wherein a hydrophobic active ingredient which is normally crystalline at ambient temperature and pressure, is, when encapsulated within the delivery system of the invention, in a non crystalline form and is thus in the latter form in the initial stage of application in an end-product. The system of the invention is therefore susceptible of releasing a normally crystalline, hydrophobic substance, in a non-crystalline form. Surprisingly, the encapsulates according to the invention were found to provide several advantages, as described below, depending in particular on the carrier material used.

It is known in the pharmaceutical industry that certain drugs or active substances can be advantageously used in a non crystalline form, in particular in an amorphous state. For instance, US 5,310,960 and US 5,310 961 describe a process for the preparation and further use of ibuprofen in an amorphous form with the effect of improving the taste of said drug. Another example is given in US 4,769,236 wherein medicaments are advantageously prepared in an amorphous form in such a way as to inhibit the formation of crystals.

However, each of these documents discloses a specific effect associated with a specific active when used in an amorphous form. Their teaching, strictly limited to the pharmaceutical field, cannot be generalised to other substances than those cited. Moreover, delivery systems are not even alluded to.

### Description of the Invention

Now, the present invention advantageously provides a novel system wherein a hydrophobic flavour or fragrance ingredient, which is crystalline at standard pressure and temperature, is in a non-crystalline form when encapsulated in such a system. A first object of the invention therefore concerns a delivery system comprising a hydrophobic flavour or fragrance ingredient or composition encapsulated in an oligomeric or polymeric carrier, said delivery system being characterised in that the flavour or fragrance ingredient or composition, when non encapsulated, is in a crystalline form at a pressure comprised between 0.5x10⁵ and 2x10⁵ Pa and a temperature comprised between 15 and 30°C, and in that, said flavour or fragrance ingredient or composition, when encapsulated in the delivery system, is in a non-crystalline form at said pressure and temperature. Therefore, the present invention provides a delivery system capable of releasing a hydrophobic, usually crystalline substance, in another physical form.

The system of the invention, in a totally unexpected manner, proved to show advantageous properties during the release of the hydrophobic flavour or fragrance ingredient or composition there-encapsulated and stabilised in a non crystalline form, and allows in particular to obtain specific effects different from those observed with a conventional encapsulation system.

A "hydrophobic ingredient or composition" designates, within the framework of the invention, an ingredient or a composition characterised by a Hildebrand solubility parameter δ below 25 [MPa]^{1/2}.

Therefore the terms "hydrophobic flavour or fragrance ingredient or composition" as used herein are deemed to define a variety of flavour and fragrance materials of both natural and synthetic origins, meeting the solubility parameter criteria above-mentioned. They include single compounds and mixtures. These ingredients are moreover characterised by the fact that they have a crystalline form in standard conditions of temperature and pressure. A non limiting list of these ingredients include heliotropine, bromelia, (5RS,6RS)-2,6,10,10-tetramethyl-1-oxaspiro-[4,5]dec-6-yl acetate, acetanisole, methylsalicylique aldehyde, *para*-ethyl phenol, phenol, phenylethyl salicylate, menthol, cyclohexanecarboxamide, veratraldehyde, xylenol, dodecanoic acid, thymol, heliotropyl acetate, methyl anisate, methylnaphtylketone, myristic acid, palmitic acid, dimethylphenol, dimethyl acrylic acid, coumarine, methyl cyclopentenolone, 7-methylcoumarin, phenylacetate, acetylpyrazine, phenylacetic acid, isoeugenyl acetate, raspberry ketone, naringin, propenyl guaethol, tetramethylpyrazine, acetylmethyl carbinol, 3-hydroxy-2-ethyl-4-pyranone, malic acid, resorcinol, benzoic acid, cinnamic acid, benjoin sumatra, benjoin siam, citric acid, tartric acid, camphor, quinine chlorohydrate, ascorbic acid, borneol, glutamic acid, 5-methyl quinoxaline and malt extract. Other examples of flavour or fragrance components suitable for the purpose of the invention may be found in the current literature, e.g. in Fenaroli's Handbook of Flavour Ingredients, 1975, CRC Press ; Synthetic Food Adjuncts, 1947 by M.B. Jacobs, edited by Van Nostrand; or Perfume and Flavour Chemicals by S. Arctander, 1969, Montclair, N.J. (USA). These substances are well known to the person skilled in the art of perfuming, flavouring and/or aromatizing consumer products, i.e. of imparting an odour and/or a flavour or taste to a consumer product traditionally perfumed or flavoured, or of modifying the odour and/or taste of said consumer products. According to an advantageous embodiment of the invention, one will use a substance selected from the group consisting of heliotropine, benzophenone, menthol, methyl anisate and propenyl guaethol. Another most advantageous embodiment involves the use of menthol.

The hydrophobic flavour or fragrance ingredient or composition used according to the invention is advantageously encapsulated and stabilised in a non crystalline form within an oligomeric or polymeric carrier. The carrier composition or the process for the preparation of the system of the invention are key factors to stabilise the flavour or fragrance ingredient or composition in a non crystalline state within the delivery system.

In a first embodiment of the invention, the encapsulated ingredient or composition, is not miscible in the encapsulating carrier material at ambient temperature, i.e. at a temperature typically comprised between 15 and 30°C.

Appropriate concentrations of flavour or fragrance ingredient or composition in the matrix as defined in this embodiment are comprised between 0.1 and 70% by weight, relative to the weight of the dried product, and preferably between 5 and 20%.

Appropriate oligomeric or polymeric carrier materials according to this embodiment of the invention are any carbohydrates or carbohydrate derivatives which can be readily processed through encapsulation techniques to form a barrier material protecting the ingredient there-encapsulated. Particular examples of suitable materials include those selected from the group consisting of sucrose, glucose, lactose, maltose, fructose, ribose, dextrose, isomalt, sorbitol, mannitol, xylitol, lactitol, maltitol, pentatol, arabinose, pentose, xylose, galactose, Trehalose^{®}, hydrogenated corn syrup, maltodextrin, modified starches such as Capsul^{®} (hydrogen octenylbutanedioate amylodextrin ; origin : National Starch, USA), agar, carrageenan, gums, polydextrose and derivatives and mixtures thereof. Other suitable carrier ingredients are cited in reference texts such as H. Scherz, Hydrokolloide: Stabilisatoren, Dickungs- und Geliermittel in Lebensmittel, Band 2 der Schriftenreihe Lebensmittelchemie, Lebensmittelqualität, Behr's VerlagGmbH & Co, Hamburg, 1996. Maltodextrin is a particularly useful such material.

The above mentioned carrier materials are given hereby by way of example and they are not to be interpreted as limiting the invention.

An emulsifier agent is optionally added to the mixture constituted by the oligomeric or polymeric component and the volatile hydrophobic flavour or fragrance ingredient. Typical examples of suitable emulsifiers include lecithin, citric acid esters of fatty acids and the mixtures of the latter with vegetable oils such as fractionated coconut oil, but other suitable emulsifiers are cited in reference texts such as Food emulsifiers and their applications, 1997, edited by G.L. Hasenhuettl and R.W. Hartel.

The delivery system of this embodiment of the invention proved to be particularly advantageous when used to encapsulate a flavour ingredient or composition. In fact, the system of the invention turned out to provide a totally unexpected boosting effect of the flavour substance there-encapsulated. In other words, the intensity of the flavour released from such a system was perceived in a very surprising intensive manner, as is well demonstrated by the examples below. Consequently, while the dosage required for perception of poorly water soluble compounds is usually high, the system of the invention advantageously allowed it to provide the same sensory effect at a much lower dosage of active flavouring component.

The delivery system of this embodiment of the invention, according to which the encapsulated ingredient is not soluble in the encapsulating carrier at ambient temperature and pressure, may be prepared by different encapsulation processes. In particular, it can have the form of a spray-dried product, or yet of an extruded product, as long as at the end of the process, the system is cooled quickly enough below the melting point of the encapsulated ingredient, thus leaving the latter in a supercooled non crystalline state. The cooling step is therefore the essential step in the process for the preparation of a delivery system according to this first embodiment of the invention.

In the case of a spray-dried powder, the process for the preparation of this product comprises the steps of dispersing the active flavour or fragrance ingredient or composition with an aqueous solution of the carrier based on a wall-forming carbohydrate material, then heating the dispersion to a temperature above the melting point of the flavour or fragrance ingredient or composition, then homogenising the dispersion and finally spray-drying the latter such that the flavour or fragrance ingredient or composition has no time to re-crystallise. The spray-drying apparatus used in such a process can be of any one of the various commercially available apparatuses. Examples of such spray-drying apparatuses are the Anhydro Dryer (origin : Anhydro Corp. of Attleboro Falls, Mass.) the Niro Dryer (manufactured by Niro Atomizer Ltd., Copenhagen, Denmark), or a Leaflash apparatus (origin : Rhône Poulenc, France).

In the case of extrusion, the process which leads to an extruded delivery system as defined above comprises the steps of dispersing the hydrophobic flavour or fragrance ingredient or composition within the carrier; extruding the dispersion at a temperature above the melting temperature of the flavour or fragrance ingredient or composition ; and cooling the extruded melt down below the melting point of the flavour or fragrance ingredient or composition at a cooling rate such that the latter has no time to re-crystallise. Therefore, at the end of the process, the active is, within the carrier material, in a non-crystalline glassy state. The process of the invention uses conventional extruding apparatuses. An example of a commercially acceptable extruding apparatus is that under the trade name designation of Clextral^{®} BC 21 twin-screw extruder. However, extruding apparatuses are not limited to the twin screw variety and may also include, for example, single screw, ram, or other similar extrusion methods. The extrusion apparatus is equipped with a temperature regulation mechanism allowing the progressive increase of the temperature of the mixture up to a value typically comprised between 80 and 130°C, at which point the melt can be extruded.

The system corresponding to the first embodiment of the invention may be easily and advantageously used in applications wherein a high intensity of a flavour ingredient or composition is desired, such as in chewing gums, hard-boiled candies or yet in compressed tablets, lozenges, bakery applications, chocolate applications, gummies, chewy sweets, or oral care products such as toothpaste.

According to another embodiment of the invention, a distinct oligomeric or polymeric carrier material will be used. The latter has the particularity of being miscible with the active ingredient to be encapsulated. The nature of the carrier allowed to provide a delivery system according to the invention, i.e. wherein the active ingredient is in a non crystalline form.

In an advantageous way of carrying out this other embodiment of the invention, the delivery system consists of an extruded product, and the carrier essentially comprises a cellulose ether which constitutes the continuous phase of the system, and the system further comprises a plasticizer miscible with said cellulose ether. Preferably, the cellulose ether is selected from the group consisting of methylcellulose, ethylcellulose, hydroxyethylcellulose, hydroxypropylcellulose, hydroxypropylmethylcellulose and hydroxypropylmethylcellulose phtalate.

While these cellulose derivatives have already been described as part of the dispersed phase of an extrudable matrix, for instance in US 6,187,351, to our knowledge they have never until today, been taught as being suitable to form the continuous phase of a granular, i.e. extruded, product. This solution may have never been considered because the known aqueous cellulose ether systems are characterised by a so-called "inverse solubility-temperature behaviour" which implies that the polymers are soluble in cold water, but phase separate upon heating above a so-called lower critical solution temperature (LCST) which is typically above 40 and 50°C (see for example Doelker 1993, in Cellulose derivates, Advances in Polymer Science, Vol. 107, pp. 199-265). Therefore aqueous cellulose ethers do not form a homogeneous thermoplastic material upon extrusion, the latter being performed typically above 40-50°C. Yet, we have now been able to solve this problem, and to extrude cellulose ethers at temperatures above 40-50°C, with the particular aim of preparing a delivery system as defined in the present invention, i.e. wherein a normally crystalline hydrophobic solid is stabilised in a non crystalline form within a delivery system. In a more general and at the same time unexpected manner, this novel carrier composition proved also to be advantageously used with hydrophobic flavour or perfume materials which are not crystalline at ambient temperature and pressure, i.e. typically liquids.

Therefore, another object of the invention is a novel granular delivery system comprising a hydrophobic flavour or fragrance ingredient or composition encapsulated in an oligomeric or polymeric carrier, characterised in that the carrier essentially consists of a cellulose ether, and in that the system comprises a plasticizer having a Hildebrand solubility parameter δ below 48 [MPa]^{1/2}.

Examples of cellulose ethers suitable for this object of the invention are defined above, and include in particular methylcellulose, ethylcellulose, hydroxyethylcellulose, hydroxypropylcellulose, hydroxypropylmethylcellulose and hydroxypropylmethylcellulose phtalate.

The cellulose ether constitutes the major polymeric constituent of the carrier. More particularly, it represents more than 50% of the oligomers and/or polymers constituting the carrier. This ingredient, when cited in the prior art, has only been disclosed as being used in the dispersed phase of an extrudable matrix composition.

The system may contain optional ingredients such as colorants, fillers or yet antioxidants which are of common use in the encapsulation field.

Moreover, an emulsifier agent is optionally added to the mixture constituted by the carrier component and the volatile flavour or fragrance ingredient. Typical examples of suitable emulsifiers include lecithin, citric acid esters of fatty acids and the mixtures of the latter with vegetable oils such as fractionated coconut oil, but other suitable emulsifiers are cited in reference texts such as Food emulsifiers and their applications, 1997, edited by G.L. Hasenhuettl and R.W. Hartel.

The plasticizer is an essential component of this novel delivery system as it makes it possible to extrude ether derivatives as carrier materials which are otherwise not extrudable. The plasticizer used in the present case is characterised by a particular Hildebrand solubility parameter, which parameter is known in the art to provide a useful polarity scale correlated to the cohesive energy density of molecules. The Handbook of Solubility Parameters (ed. A.F.M. Barton, CRC Press, Bocca Raton, 1991) gives a list of δ values for many chemicals, as well as recommended group contribution methods allowing to calculate δ values for complex chemical structures. Suitable plasticizers for the purpose of the invention include in particular, propylene glycol, glucose, isomalt, corn starch syrup, glycerol, ethylene glycol, dipropylene glycol, butylene glycol, triacetin, trialkylcitrate, triethylcitrate, polyethylene glycol, polypropylene glycol and organic acids or mixtures thereof. In a particular embodiment of the invention, the hydrophobic active ingredient to be encapsulated can itself act as a plasticizer.

The proportion of plasticizer in the delivery system varies between 5 and 95 % by weight relative to the total weight of the composition.

We have unexpectedly found that the system comprising a hydrophobic flavour or fragrance ingredient or composition, a plasticizer as defined above and the cellulose ether, advantageously avoids a phase separation of the ingredients during the extrusion.

While this novel combination of carrier and plasticizer can be used with any kind of hydrophobic flavour or fragrance material, it can be in particular advantageously used for the encapsulation of active ingredients as defined above for the purpose of the present invention, i.e. for hydrophobic flavour or fragrance ingredients or compositions which are crystalline at standard temperature and pressure and which it is desired to produce in another physical form. In fact, this carrier-plasticizer combination, when extruded with such ingredients, provides a flavour or fragrance delivery system wherein the active ingredient is in a non crystalline form.

The final product has the advantage of being a free flowing dust-free granule with a reduced surface to volume ratio, which minimises the dissolution rate of modified celluloses, especially in hot water.

The use of cellulose derivatives is also advantageous compared with usual carrier materials used in extrusion processes, as it allows to maximise the flavour or fragrance retention upon extrusion as shown in the examples below.

In a more particular embodiment, the system described above consists of menthol as flavour ingredient, hydroxypropylmethylcellulose (HPMC) as carrier material, and propylene glycol as plasticizer. In fact, the latter system proved to be particularly efficient and to provide a very long lasting taste of menthol when consumed. Moreover, the concentration of menthol which can be used in such a system, can advantageously go up to 60% by weight, relative to the dried weight of the product.

In another particular embodiment, the active ingredient used within the same system as that above mentioned, namely HPMC and propylene glycol, is a perfuming ingredient.

The encapsulation system of the embodiment of the invention wherein the encapsulated ingredient is miscible with the encapsulating material at ambient temperature and pressure may be prepared by a standard extrusion process comprising the basic steps of combining and blending a hydrophobic flavour or fragrance ingredient or composition with a so-called "matrix" comprising a cellulose derivative and a plasticizer as defined above, under temperature and stirring conditions useful to produce a uniform melt thereof ; extruding the uniform melt through a die ; and chopping, cutting, grinding or pulverising the material obtained as it exits the die or after cooling the melt.

The extruding apparatuses suitable for the process are of the same type as those described above and the various parameters of the process may be adapted by a skilled person in the art.

All the delivery systems here-above disclosed, in any one of the described embodiments, can be advantageously used to improve, enhance or modify the organoleptic properties of a perfuming or flavouring composition, when they are added to such a composition. In the field of flavours, these consumer products may include foods, beverages, pharmaceuticals and the like. More particularly, the system of the invention may be advantageously used to flavour chewing gums, hard-boiled candies, compressed tablets, lozenges, bakery applications, chocolate applications, gummies, chewy sweets, or oral care products such as toothpaste. On the other hand, in the field of perfumery, the granular solids according to the invention may be advantageously incorporated in a perfuming composition to be added to functional products such as detergents or fabric softeners. Other functional perfumery applications such as soaps, bath or shower gels, deodorants, body lotions, shampoos or other hair-care products, household cleaners, cleaning and deodorising blocks for toilet tanks may constitute suitable applications for the products according to the invention. These examples are of course neither exhaustive, nor restrictive of the invention.

The concentrations in which the delivery systems of the invention can be incorporated in such consumer products vary in a wide range of values, which are dependent on the nature of the product to be flavoured or perfumed and on the release effect desired. Typical concentrations, to be taken strictly by way of example, are comprised in a range of values as wide as from a few ppm up to 5 or 10% by weight relative to the weight of the flavouring or perfuming composition or finished consumer product into which they are incorporated.

The invention will now be illustrated by way of the following examples, but is not limited to these examples. Temperatures are given in degrees centigrade and abbreviations have the meaning common in the art.

### Description of figures

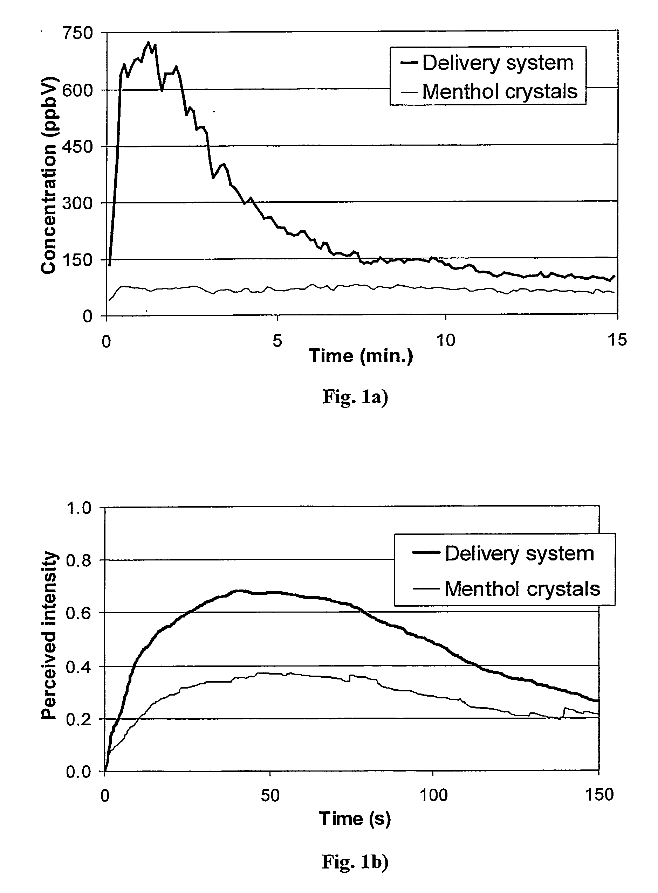

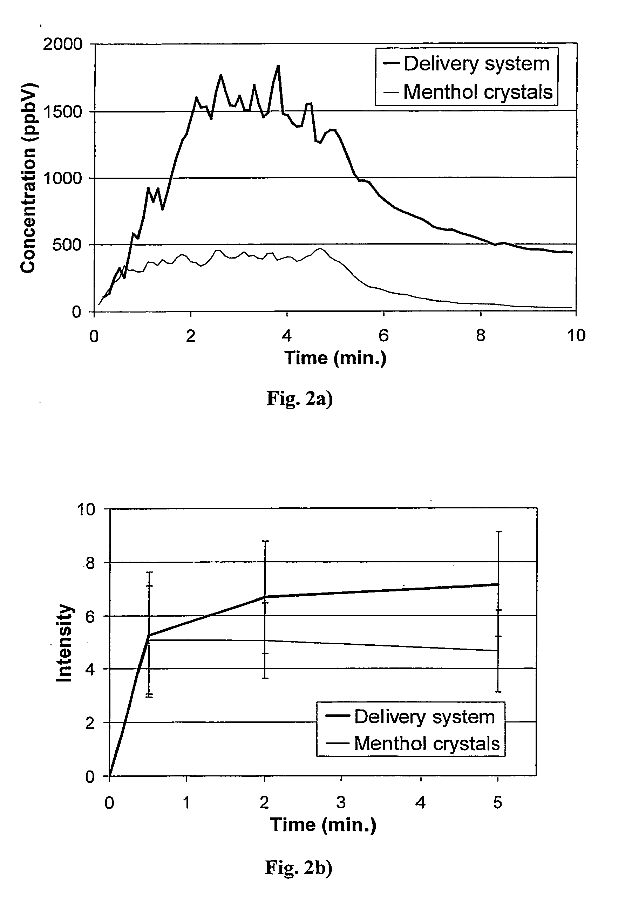

Fig. 1a) and Fig. 2a) compare panelists' exhalation of menthol from a chewing gum, respectively a hard-boiled candy when the flavour is incorporated in the application in the form of a delivery system according to the invention and when it is incorporated in the form of menthol crystals.
Fig. 1b) and 2b) compare, in the same respective applications than in Fig. 1a) and 2a), the intensity of the flavour perceived by a panel as a function of time, when the flavour is incorporated in the application in the form of a delivery system according to the invention and when it is incorporated in the form of menthol crystals.

### Embodiments of the Invention

### Example 1

A flavoured delivery system was prepared by admixing the following ingredients :

| Ingredients | Parts by weight |
|---|---|
| Menthol ¹⁾ | 10 |
| Water | 7 |
| Maltodextrin 19 DE | 82 |
| 1:1 Mixture of Citrem^{®2)}/fractionated coconut oil ³⁾ | 1 |
| Total | 100 |

| | |
|---|---|
| 1) origin : Firmenich SA, Geneva, Switzerland 2) origin : Danisco, Denmark 3) origin: Stearinerie-Dubois | |

Using a Brabender (DDW-P3-DDSR20N) gravimetric feeder, a spray-dried formulation containing the menthol, water and maltodextrin was fed into a bench-top 16 mm Eurolab Thermoprism twin-screw extruder, equipped with a screw configuration containing only transport elements. This spray-dried powder was conveyed through barrel 1 which was kept at ambient temperature. The mixture of Citrem^{®} and fractionated coconut oil was injected (peristaltic pump : Watson Marlow 505 S) in barrel 2 at 30°. The powder was further heated up to 80° in barrel 3 and then to 110° in barrel 4 and 5 and extruded through a 2 mm die hole and further cooled at a cooling rate such that the menthol had no time to re-crystallise as proofed by the thermal analysis here-below. At a screw speed of 130 rpm, and an overall through put of 0.5 kg/h, the mass temperature and pressure measured in the front plate of the extruder were of 104° and 15x10⁵ Pa, respectively.

The product obtained was a granular delivery system. A thermal analysis was carried out by DSC (Differential Scanning Calorimetry) on said granular delivery system on the one hand, and on crystalline menthol on the other.

Temperature programme :
1) isotherm at -20° during 4 min
2) temperature slope from -20° to 95° at 10°/min
3) cooling from 95° to -20° at 20°/min
4) isotherm at -20° during 4 min
5) temperature slope from -20° to 95° at 10°/min

The analysis displayed that menthol was in a non-crystalline form after the encapsulation process above-described. The same analysis carried out on menthol as such revealed a re-crystallisation of the latter with formation of polymorphic forms, as a function of the cooling rate.

### Example 2

### Comparison between applications flavoured with the delivery system of Example 1 and applications flavoured with menthol crystals

A chewing gum base was prepared by blending crystalline sorbitol (54.8% of the final blend), acesulfame K (0.1%) and aspartame (0.1%) in a Turbula blender. Half the blend was mixed with a pre-warmed Nova T gum base (22.0%) (origin : L.A. Dreyfus) in a Winkworth sigma-blade mixer at 50-55° for 2 min. The remaining powder blend was then added with a humectant syrup (Lycasin^{®} 80/55 (12%), Sorbi^{®} (6%), glycerin (4%)) and mixed for a further 7 min.

To this preparation, a delivery system as described in Example 1 was dosed at 2.07% and mixed for 1 min. To another sample of the same preparation, menthol crystals (957789, origin : Firmenich SA, Geneva, Switzerland) were added at a dosage of 0.18% and mixed for 1 min.

On the other hand, a hard-boiled candy base was prepared by adding 30 g of glucose syrup 42 DE to 122 g of sucrose syrup 65 Brix. The mixture was heated to 148° in a copper pan. At 148°, the copper pan was removed and the temperature checked. When temperature reached 135°, the flavouring system, namely a delivery system according to Example 1 dosed at 1% on the one hand, and menthol crystals dosed at 0.12% on the other, was added. The cooked mass was then poured at ambient temperature (at less than 40% rH) in appropriate Teflon^{®} molds.

A panel tested the delivery system of the invention compared with menthol crystals in the chewing-gum application on the one hand and in the hard-boiled candy application on the other.
Fig. 1a) and 2a) report the concentration of menthol exhaled by the panelists as a function of time, measured by an Affirm^{®} method.

### Affirm^{®} Protocol :

Panelists rinsed their mouth with water. A blank mouthspace level with the lips sealed round the AFFIRM^{®} mouthpiece was recorded; the panelists inhaling through the nose and exhaling through the mouth for one minute. Measurements of the menthol and acetone signals were recorded during 15 minutes while the panelists were chewing (for chewing gum). For hard-boiled candy, measurements were recorded during 10 minutes.
Fig. 1b) reports the perceived intensity of menthol as a function of time in the case of the chewing gum application. More particularly fifteen panellists were asked to evaluate on a continuous manner the menthol intensity over time using a computer acquisition system. Panellists scored the perceived intensity by moving the cursor from an electronic mouse on a linear intensity scale varying from 0 to 1.
Fig. 2b) which corresponds to the hard boiled candy reports the results of a sensory test performed on a longer time scale using a discontinuous technique. Fifteen trained panellists had to evaluate the menthol intensity of the application at specific periods of time, namely 30 s, 2 min and 5 min, while sucking, on a linear intensity scale varying from 0 to 10.

It appears from the results given in Fig. 1a) and 2a) that the concentration in menthol exhaled is increased with the delivery system of the invention, compared with the concentration exhaled when menthol crystals are used as such in the same application. On the other hand, Fig. 1b) and 2b) show that the perceived intensity as a function of time is also increased with the delivery system of the invention, compared with that perceived when menthol crystals are used as such in the tested applications.

### Example 3

A flavour delivery system was prepared by admixing the following ingredients :

| Ingredient | Parts by weight |
|---|---|
| Menthol ¹⁾ | 40 |
| Propylene glycol | 19 |
| Hydroxypropylmethylcellulose ²⁾ | 40 |
| 1:1 Mixture of Citrem^{® 3)}/ fractionated coconut oil ⁴⁾ | 1 |
| Total | 100 |

| | |
|---|---|
| 1) Firmenich menthol Nat. N° 957759; origin : Firmenich SA, Geneva, Switzerland 2) origin : Dow chemicals 3) origin : Danisco, Denmark 4) origin : Stearinerie-Dubois | |

Using a Brabender (DDW-P3-DDSR20N) gravimetric feeder, a dry blend of hydroxypropylmethylcellulose and menthol was fed into a bench-top 16 mm Eurolab Thermoprism twin-screw extruder, equipped with a screw configuration containing only transport elements. This powder mixture was conveyed through barrel 1 which was kept at ambient temperature. The mixture of Citrem^{®} and fractionated coconut oil was injected (peristaltic pump : Watson Marlow 505 S) in barrel 2 at 30°. Menthol was molten in barrel 3 at 60° and propylene glycol was injected (peristaltic pump : Watson Marlow 505 S) in barrel 4 at 90°. The material was further heated up to 120° in barrel 5 and extruded through a 2 mm die hole. At a screw speed of 130 rpm, and an overall through put of 0.5 kg/h, the mass temperature and pressure measured in the front plate of the extruder were of 116° and 11x10⁵ Pa, respectively.

The granular delivery system thus obtained presented a very high load in flavour, as well as free-flowing characteristics, without any caking. A thermal analysis was carried out by DSC, following the same temperature programme as that described in Example 1. The first temperature slope allowed to melt the menthol. The cooling (step 3) usually leads to a re-crystallisation of the latter. However, in that case, no re-crystallisation was observed, even at -20°. The second temperature slope allowed to verify that menthol was non-crystalline, as no fusion peak was observed.

### Example 4

A flavour delivery system was prepared by admixing the following ingredients :

| Ingredients | Parts by weight |
|---|---|
| Orange ¹⁾ | 7 |
| Propylene glycol | 54 |
| Hydroxypropylmethylcellulose ²⁾ | 38 |
| 1:1 Mixture of Citrem^{® 3)}/fractionated coconut oil ⁴⁾ | 1 |
| Total | 100 |

| | |
|---|---|
| 1) Firmenich orange flavour N° 51941 ; origin : Firmenich SA, Geneva, Switzerland 2) origin: Dow chemicals 3) origin : Danisco, Denmark 4) origin: Stearinerie-Dubois | |

Using a Brabender (DDW-P3-DDSR20N) gravimetric feeder, a dry blend of hydroxypropylmethylcellulose and orange oil was fed into a bench-top 16 mm Eurolab Thermoprism twin-screw extruder, equipped with a screw configuration containing only transport elements. This dry blend was conveyed through barrel 1 which was kept at ambient temperature. The mixture of Citrem^{®} and fractionated coconut oil was injected (peristaltic pump : Watson Marlow 505 S) in barrel 2 at 30°. The dry blend was then heated to 60° in barrel 3 and propylene glycol was injected (peristaltic pump : Watson Marlow 505 S) in barrel 4 at 90°. The material was further heated up to 120° in barrel 5 and extruded through a 2 mm die hole. At a screw speed of 130 rpm, and an overall through put of 0.5 kg/h, the mass temperature and pressure measured in the front plate of the extruder were of 125° and 32x10⁵ Pa, respectively.

The granular delivery system obtained was free-flowing and did not present any caking, thus bearing evidence of the efficiency of the matrix composition encapsulating a liquid hydrophobic active

### Example 5

A flavour delivery system was prepared by admixing the following ingredients :

| Ingredient | Parts by weight |
|---|---|
| Limonene | 50 |
| Ethylcellulose ¹⁾ | 50 |
| Total | 100 |

| | |
|---|---|
| 1) Aqualon^{®} ; origin : Hercules | |

The process used for the preparation of the delivery system is similar to that described in example 4. In the present case, limonene was used at the same time as flavouring ingredient, and as a plasticizer. The obtained particles were soft and non-sticking.
In an aqueous environment, the particles proved to be characterised by a water absorption of 2% at 50% rH during 24 h.

Similar products, namely particles with same characteristics were obtained when replacing limonene by an equimolar mixture of Verdox® (2-tert-butyl-1-cyclohexyl acetate; origin: International Flavors and Fragrances, USA), allyl heptanoate, hexyl salicylate and phenoxy isobutyrate.

## Claims

1. A delivery system obtainable by extrusion comprising a hydrophobic flavour or fragrance ingredient or composition encapsulated in an oligomeric or polymeric carrier, **characterised in that** more than 50% by weight of the carrier essentially consists of a cellulose ether and **in that** the system comprises a plasticizer having a Hildebrand solubility parameter δ below 48 [MPa]^{1/2}.

2. A delivery system according to claim 1, **characterised in that** the cellulose ether is selected from the group consisting of methylcellulose, ethylcellulose, hydroxyethylcellulose, hydroxypropylcellulose, hydroxypropylmethylcellulose and hydroxypropylmethylcellulose phtalate.

3. A delivery system according to claim 1, **characterised in that** the plasticizer is selected from the group consisting of propylene glycol, glucose, isomalt, corn starch syrup, glycerol, ethylene glycol, dipropylene glycol, butylene glycol, triacetin, trialkylcitrate, triethylcitrate, polyethylene glycol, polypropylene glycol and organic acids.

4. A delivery system according to claim 3, **characterised in that** the cellulose ether consists of hydroxypropylmethylcellulose.

5. A delivery system according to any one of claims 1 to 4, **characterized in that** the flavour or fragrance ingredient is selected from the group consisting of heliotropine, bromelia, (5RS,6RS)-2,6,10,10-tetramethyl-1-oxaspiro-[4,5]dec-6-yl acetate, acetanisole, methylsalicylique aldehyde, *para*-ethyl phenol, phenol, phenylethyl salicylate, menthol, cyclohexanecarboxamide, veratraldehyde, xylenol, dodecanoic acid, thymol, heliotropyl acetate, methyl anisate, methylnaphtylketone, myristic acid, palmitic acid, dimethylphenol, dimethyl acrylic acid, coumarine, methyl cyclopentenolone, 7-methylcoumarin, phenylacetate, acetylpyrazine, phenylacetic acid, isoeugenyl acetate, raspberry ketone, naringin, propenyl guaethol, tetramethylpyrazine, acetylmethyl carbinol, 3-hydroxy-2-ethyl-4-pyranone, malic acid, resorcinol, benzoic acid, cinnamic acid, benjoin sumatra, benjoin siam, citric acid, tartric acid, camphor, quinine chlorohydrate, ascorbic acid, borneol, glutamic acid, 5-methyl quinoxaline and malt extract.

6. A delivery system according to any one of claims 1 to 4, **characterised in that** the flavour or fragrance ingredient is selected from the group consisting of heliotropine, benzophenone, menthol, methyl anisate and propenyl guaethol.

7. A delivery system according to claim 4, **characterised in that** the flavour ingredient consists of menthol.

8. A delivery system according to claim 7, **characterised in that** it comprises up to 60% by weight relative to the dried weight of the system, of menthol.

9. A flavoured product or composition **characterised in that** it comprises as an active ingredient, a delivery system according to any one of claims 1 to 8.

10. A flavoured product or composition according to claim 9 in the form of a chewing gum, a hard-boiled candy, a compressed tablet, a lozenge, a bakery application, a chocolate application, a gummy, a chewy sweet, or an oral care product.

11. A perfumed product or article **characterised in that** it comprises as an active ingredient, a delivery system according to any one of claims 1 to 8.

12. A perfumed product or article according to claim 11 in the form of a detergent, a fabric softener, a soap, a bath or shower gel, a deodorant, a body lotion, a shampoo or other hair-care product, a household cleaner or a cleaning or deodorising block for toilet tanks.

13. A method to improve, enhance or modify the organoleptic properties of a perfuming or flavouring composition, **characterised in that** there is added to such composition a delivery system according to any one of claims 1 to 8.

14. A process for the preparation of a delivery system according to claim 1, comprising the steps of :
a) combining and blending the flavour or fragrance ingredient or composition with the carrier to form a uniform melt ;
b) extruding said melt through a die ; and
c) chopping, cutting, grinding or pulverising the material obtained as it exits the die or after cooling the melt.

## Patentansprüche

1. Abgabesystem, erhältlich durch Extrusion, umfassend einen hydrophoben Aroma- oder Duftbestandteil oder eine Zusammensetzung, eingekapselt in einem oligomeren oder polymeren Träger, **dadurch gekennzeichnet, daß** mehr als 50 Gew.-% des Trägers im wesentlichen aus einem Celluloseether bestehen und daß das System einen Weichmacher mit einem Hildebrand-Löslichkeitsparameter □ unter 48 [MPa]^{1/2} umfaßt.

2. Abgabesystem gemäß Anspruch 1, **dadurch gekennzeichnet, daß** der Celluloseether aus der Gruppe, bestehend aus Methylcellulose, Ethylcellulose, Hydroxyethylcellulose, Hydroxypropylcellulose, Hydroxypropylmethylcellulose und Hydroxypropylmethylcellulosephthalat, ausgewählt ist.

3. Abgabesystem gemäß Anspruch 1, **dadurch gekennzeichnet, daß** der Weichmacher aus der Gruppe, bestehend aus Propylenglycol, Glucose, Isomalt, Maisstärkesirup, Glycerol, Ethylenglycol, Dipropylenglycol, Butylenglycol, Triacetin, Trialkylcitrat, Triethylcitrat, Polyethylenglycol, Polypropylenglycol und organischen Säuren, ausgewählt ist.

4. Abgabesystem gemäß Anspruch 3, **dadurch gekennzeichnet, daß** der Celluloseether aus Hydroxypropylmethylcellulose besteht.

5. Abgabesystem gemäß einem der Ansprüche 1 bis 4, **dadurch gekennzeichnet, daß** der Aroma- oder Duftbestandteil aus der Gruppe, bestehend aus Heliotropin, Bromelia, (5RS,6RS)-2,6,10,10-Tetramethyl-1-oxaspiro-[4,5]dec-6-yl-acetat, Acetanisol, Methylsalicylaldehyd, para-Ethylphenol, Phenol, Phenylethylsalicylat, Menthol, Cyclohexancarboxamid, Veratrumaldehyd, Xylenol, Dodecansäure, Thymol, Heliotropylacetat, Methylanisat, Methylnaphtylketon, Myristinsäure, Palmitinsäure, Dimethylphenol, Dimethylacrylsäure, Cumarin, Methylcyclopentenolon, 7-Methylcumarin, Phenylacetat, Acetylpyrazin, Phenylessigsäure, Isoeugenylacetat, Himbeerketon, Naringin, Propenylguaethol, Tetramethylpyrazin, Acetylmethylcarbinol, 3-Hydroxy-2-ethyl-4-pyranon, Äpfelsäure, Resorcinol, Benzoesäure, Zimtsäure, Gummi Benzoe Sumatra, Gummi Benzoe Siam, Zitronensäure, Weinsäure, Campher, Chininchlorhydrat, Ascorbinsäure, Borneol, Glutaminsäure, 5-Methylchinoxalin und Malzextrakt, ausgewählt ist.

6. Abgabesystem gemäß einem der Ansprüche 1 bis 4, **dadurch gekennzeichnet, daß** der Aroma- oder Duftbestandteil aus der Gruppe, bestehend aus Heliotropin, Benzophenon, Menthol, Methylanisat und Propenylguaethol, ausgewählt ist.

7. Abgabesystem gemäß Anspruch 4, **dadurch gekennzeichnet, daß** der Aromabestandteil aus Menthol besteht.

8. Abgabesystem gemäß Anspruch 7, **dadurch gekennzeichnet, daß** es bis zu 60 Gew.-%, relativ zu dem Trockengewicht des Systems, von Menthol umfaßt.

9. Aromatisiertes Produkt oder Zusammensetzung, **dadurch gekennzeichnet, daß** es als aktiven Bestandteil ein Abgabesystem gemäß einem der Ansprüche 1 bis 8 umfaßt.

10. Aromatisiertes Produkt oder Zusammensetzung gemäß Anspruch 9 in der Form eines Kaugummis, eines hartgekochten Bonbons, einer gepreßten Tablette, einer Pastille, einer Bäckereianwendung, einer Schokoladenanwendung, eines Gummis, einer kaubaren Süßigkeit oder eines Mundpflegeprodukts.

11. Parfümiertes Produkt oder Gegenstand, **dadurch gekennzeichnet, daß** es als aktiven Bestandteil ein Abgabesystem gemäß einem der Ansprüche 1 bis 8 umfaßt.

12. Parfümiertes Produkt oder Gegenstand gemäß Anspruch 11 in der Form eines Detergens, eines Weichspülers, einer Seife, eines Bade- oder Duschgels, eines Deodorants, einer Körperlotion, eines Shampoo oder anderen Haarpflegeprodukts, eines Haushaltsreinigers oder eines reinigenden oder desodorierenden Blocks für Spülkästen.

13. Verfahren zum Verbessern, Verstärken oder Modifizieren der organoleptischen Eigenschaften einer parfümierenden oder aromatisierenden Zusammensetzung, **dadurch gekennzeichnet, daß** zu einer derartigen Zusammensetzung ein Abgabesystem gemäß einem der Ansprüche 1 bis 8 hinzugegeben wird.

14. Verfahren für die Herstellung eines s Abgabesystems gemäß Anspruch 1, umfassend die Schritte:
a) Vereinigen und Mischen des Aroma- oder Duftbestandteils oder der Zusammensetzung mit dem Träger, um eine gleichmäßige Schmelze zu erzeugen;
b) Extrudieren der Schmelze durch eine Düse; und
c) Zerhacken, Zerschneiden, Zermahlen oder Pulverisieren des erhaltenen Materials, wenn es die Düse verläßt, oder nach dem Abkühlen der Schmelze.

## Revendications

1. Système de relargage susceptible d'être obtenu par extrusion, comprenant un ingrédient ou une composition d'arôme ou de fragrance hydrophobe encapsulé(e) dans un véhicule oligomérique ou polymérique, **caractérisé en ce que** plus de 50% en poids du véhicule consiste essentiellement en un éther de cellulose et **en ce que** le système comprend un plastifiant ayant un paramètre de solubilité de Hildebrand □ inférieur à 48 [MPa]^{1/2}.

2. Système de relargage selon la revendication 1, **caractérisé en ce que** l'éther de cellulose est choisi dans le groupe consistant en la méthylcellulose, l'éthylcellulose, l'hydroxyéthylcellulose, l'hydroxypropylcellulose, l'hydroxypropylméthylcellulose et le phtalate d'hydroxypropylméthylcellulose.

3. Système de relargage selon la revendication 1, **caractérisé en ce que** le plastifiant est choisi dans le groupe consistant en le propylèneglycol, le glucose, l'isomalt, le sirop d'amidon de maïs, le glycérol, l'éthylèneglycol, le dipropylèneglycol, le butylèneglycol, la triacétine, le citrate de trialkyle, le citrate de triéthyle, le polyéthylèneglycol, le polypropylèneglycol et les acides organiques.

4. Système de relargage selon la revendication 3, **caractérisé en ce que** l'éther de cellulose consiste en l'hydroxypropylméthylcellulose.

5. Système de relargage selon l'une quelconque des revendications 1 à 4, **caractérisé en ce que** l'ingrédient d'arôme ou de fragrance est choisi dans le groupe consistant en l'héliotropine, la bromélie, l'acétate de (5RS,6RS)-2,6,10,10-tétraméthyl-1-oxaspiro-[4,5]déc-6-yle, l'acétanisole, l'aldéhyde méthylsalicylique, le para-éthylphénol, le phénol, le salicylate de phényléthyle, le menthol, le cyclohexanecarboxamide, le vératraldéhyde, le xylénol, l'acide dodécanoïque, le thymol, l'acétate d'héliotropyle, l'anisate de méthyle, la méthylnaphtylcétone, l'acide myristique, l'acide palmitique, le diméthylphénol, l'acide diméthylacrylique, la coumarine, la méthylcyclopenténolone, la 7-méthylcoumarine, l'acétate de phényle, l'acétylpyrazine, l'acide phénylacétique, l'acétate d'isoeugényle, la cétone de framboise, la naringine, le propénylguaéthol, la tétraméthylpyrazine, l'acétylméthylcarbinol, la 3-hydroxy-2-éthyl-4-pyranone, l'acide malique, le résorcinol, l'acide benzoïque, l'acide cinnamique, le benjoin de Sumatra, le benjoin du Siam, l'acide citrique, l'acide tartrique, le camphre, le chlorhydrate de quinine, l'acide ascorbique, le bornéol, l'acide glutamique, la 5-méthylquinoxaline et l'extrait de malt.

6. Système de relargage selon l'une quelconque des revendications 1 à 4, **caractérisé en ce que** l'ingrédient d'arôme ou de fragrance est choisi dans le groupe consistant en l'héliotropine, la benzophénone, le menthol, l'anisate de méthyle et le propénylguaéthol.

7. Système de relargage selon la revendication 4, **caractérisé en ce que** l'ingrédient d'arôme consiste en le menthol.

8. Système de relargage selon la revendication 7, **caractérisé en ce qu'**il comprend jusqu'à 60% en poids par rapport au poids séché du système, de menthol.

9. Produit ou composition aromatisée, **caractérisé en ce qu'**il comprend en tant qu'ingrédient actif un système de relargage selon l'une quelconque des revendications 1 à 8.

10. Produit ou composition aromatisée selon la revendication 9 sous la forme d'une gomme à mâcher, d'un bonbon dur, d'un comprimé compressé, d'une pastille à sucer, d'une application de boulangerie, d'une application de chocolat, d'un bonbon gélifié, d'une sucrerie caoutchouteuse, ou d'un produit de soin oral.

11. Produit ou article parfumé, **caractérisé en ce qu'**il comprend en tant qu'ingrédient actif un système de relargage selon l'une quelconque des revendications 1 à 8.

12. Produit ou article parfumé selon la revendication 11 sous la forme d'un détergent, d'un adoucissant pour textile, d'un savon, d'un gel de bain ou de douche, d'un déodorant, d'une lotion pour le corps, d'un shampoing ou d'un autre produit de soin capillaire, d'un nettoyant ménager ou d'un bloc nettoyant ou désodorisant pour les cuves des toilettes.

13. Procédé pour améliorer, intensifier ou modifier les propriétés organoleptiques d'une composition parfumante ou aromatisante, **caractérisé en ce qu'**on a ajouté à cette composition un système de relargage selon l'une quelconque des revendications 1 à 8.

14. Procédé de préparation d'un système de relargage selon la revendication 1, comprenant les étapes de:
a) combinaison et mélange de l'ingrédient ou de la composition d'arôme ou de fragrance avec le véhicule pour former une masse fondue uniforme;
b) extrusion de ladite masse fondue à travers une filière; et
c) hachage, découpage, broyage ou pulvérisation de la matière obtenue au fur et à mesure qu'elle sort de la filière ou après refroidissement de la masse fondue.
